# EUROPEAN PATENT APPLICATION

(11) **EP 4 821 529 A1**
(43) Date of publication of application: **14.10.2026**
(21) Application number: 24899650.6
(22) Date of filing: 22.11.2024
(51) Int. Cl.: A61M 16/06

(54) **HEADBAND ASSEMBLY AND MASK SYSTEM**

(30) Priority: 07.12.2023 CN 202311675657
(71) Applicant: BMC Medical Co., Ltd., Beijing 100089 (CN)
(72) Inventor: WANG, Yajie, Beijing 100089 (CN); LIU, Lina, Beijing 100089 (CN); HE, Long, Beijing 100089 (CN); ZHOU, Mingzhao, Beijing 100089 (CN); ZHUANG, Zhi, Beijing 100089 (CN)
(74) Representative: Grünecker Patent- und Rechtsanwälte PartG mbB
(86) International application number: PCT/CN2024/133973
(87) International publication number: WO 2025/119009

(57) **Abstract**

Disclosed are a headgear assembly and a mask system, the headgear assembly including a primary fixing part and a secondary fixing part, where the secondary fixing part is connected above the primary fixing part, the primary fixing part is configured to wrap around the back of the head of a human body, and the secondary fixing part is configured to wrap around the top of the head of the human body; where the primary fixing part includes: a plurality of fixing straps, at least two of the plurality of fixing straps being connected to each other, a free end of each of the fixing straps being provided with a connection point (9), and the connection point (9) being configured for assembly to the mask system; the secondary fixing part includes: two top headstraps spaced side by side, with free ends of the two top headstraps being connected by a fixing structure; and the plurality of fixing straps include an upper headstrap (3), an intermediate connecting strap and a lower headstrap (4), two end portions of the intermediate connecting strap being respectively connected to a middle region of each of the upper headstrap (3) and the lower headstrap (4). This allows for a comfortable and secure wear, a simple and cost-effective manufacturing process, a wide range of applicable mask types, and many other advantages.

## Description

### Cross Reference to Related Application

The present application claims the right of priority for Chinese patent application no. 202311675657.3, filed with the China National Intellectual Property Administration on December 7, 2023 and entitled "HEADGEAR ASSEMBLY AND MASK SYSTEM", which is incorporated herein by reference in its entirety.

### Technical Field

The present application relates to the technical field of ventilation therapy, and in particular to a headgear assembly and a mask system.

### Background Art

A mask system refers to a device for delivering oxygen to a user or providing positive pressure ventilation support, or for inhalation therapy. The mask system is typically composed of a mask that covers the mouth and nose region, a pipe that connects the mask to an oxygen source or a ventilator, and a headgear assembly that locates and secures the mask in a suitable position on the patient's face. As the heads of different individuals vary greatly, the headgear assembly is required to provide good comfort, conformability and sufficient fittability to ensure effective conditioning, improvement and therapeutic effects of the mask system when worn by the user.

The current headgear assembly is generally a "four-point" type, that is, it is connected to a mask of a mask system via four connection points to secure the mask to the patient's face. The "four-point" headgear assembly is mainly divided into an "I-shaped" headgear assembly and a "crown-shaped" headgear assembly, where the "I-shaped" headgear assembly has poor wearing stability, cannot be compatible with the top of the user's head, and is only paired with a mask system featuring a forehead support; while the "crown-shaped" headgear assembly is more robust than the "I-shaped" headgear assembly and can be applied to mask systems with and without a forehead support, but it requires a large amount of clinical data support on the top of the head to carry out individualized 3D modeling. Moreover, it is extremely difficult to achieve an appropriate 3D shape, and the manufacturing process is complex and costly.

### Summary

In view of the above-mentioned problems, one of the objectives of the present invention is to provide a headgear assembly, so as to achieve a comfortable and secure wear, a simple and cost-effective manufacturing process, a wide range of applicable mask types, etc., and a second objective of the present invention is to provide a mask system.

A first aspect of the present invention provides a headgear assembly, with a technical solution adopted as follows:
a headgear assembly, including a primary fixing part and a secondary fixing part, the secondary fixing part being connected above the primary fixing part, the primary fixing part being configured to wrap around the back of the head of a human body, and the secondary fixing part being configured to wrap around the top of the head of the human body; where
the primary fixing part includes:
   a plurality of fixing straps, at least two of the plurality of fixing straps being connected to each other, and a free end of each of the fixing straps being provided with a connection point for assembly to a mask system; and
   the secondary fixing part includes:
      two top headstraps spaced side by side, with free ends of the two top headstraps being connected by a fixing structure;
      where the plurality of fixing straps include:
         an upper headstrap, an intermediate connecting strap, and a lower headstrap, two end portions of the intermediate connecting strap being respectively connected to a middle region of each of the upper headstrap and the lower headstrap.

Since both the primary fixing part and the secondary fixing part are configured to position and secure the mask of the mask system in a relatively comfortable position on the face, it is ensured that the mask does not loosen or fall off during use, thereby providing a good seal and achieving the conditioning, improvement and therapeutic effects of the mask system. The primary fixing part and the secondary fixing part are arranged from the bottom to the top of the head of the human body. The primary fixing part is designed to surround the back of the head of the human body, and the secondary fixing part is designed to surround the top of the head of the human body to achieve a 3D wrapping effect of the head.

As one of the preferred solutions, the fixing structure includes a fixed connector and an adjustable connector cooperating with each other, the fixed connector being disposed on one of the two top headstraps, and the adjustable connector being disposed on the other of the two top headstraps.

The arrangement of the fixed connector and the adjustable connector may change the size of a fully enclosing structure formed by connecting the two top headstraps to adjust the tightness of the secondary fixing part to fit different head sizes.

As one of the preferred solutions, the two top headstraps have different strap lengths, and the fixed connector is disposed on the top headstrap with a shorter strap length.

As one of the preferred solutions, the fixed connector includes a buckle, and the adjustable connector includes a hook-and-loop fastener.

As one of the preferred solutions, an angle between each of the two top headstraps and the fixing strap correspondingly connected thereto ranges from 80° to 100°.

As one of the preferred solutions, angles between the two top headstraps and the fixing straps correspondingly connected thereto are equal.

In this implementation, the first top headstrap and the second top headstrap may be symmetrically distributed after being connected, such that the pressure around the head is balanced, so as to avoid applying excessive pressure or causing discomfort on specific parts, which is conducive to providing a more comfortable wearing experience. An acute-angled design reduces the gap between the headgear assembly and the head, which ensures that the headgear assembly fits snugly against the head and prevents displacement or loosening of the headgear assembly during use, thereby enhancing the overall performance and wearing experience of the headgear assembly.

As one of the preferred solutions, the intermediate connecting strap includes a left headstrap and a right headstrap arranged obliquely relative to each other, and the left headstrap and the right headstrap together with the upper headstrap and the lower headstrap enclose a variable-diameter wrapping cavity.

The variable-diameter wrapping cavity can be understood to have different inner diameters or diameters in the height direction. As the area of the wrapping cavity varies from top to bottom to fit the curvature and shape variations of the back of the head of the human body, which can achieve a better fitting and wrapping effect, reduce the overall weight, and provide the users with a more comfortable wearing experience.

As one of the preferred solutions, the lower headstrap curves toward a neck region of the human body to form an arc-shaped opening with an obtuse angle.

In this embodiment, the lower headstrap bends at its central region, forming an inverted V-shaped structure with an opening facing the neck of the human body. An angle of the opening is an obtuse angle, facilitating two mutually symmetrical sides of the V-shaped lower headstrap to extend obliquely downward toward the neck of the human body, and can thus go around behind the ears when assembled with the mask, thereby reaching the neck region of the human body to achieve a 3D wrapping effect.

As one of the preferred solutions, the plurality of fixing straps and the two top headstraps have planar features.

As one of the preferred solutions, the two top headstraps are connected to the upper headstrap, and are equidistant from the intermediate connecting strap.

As one of the preferred solutions, the primary fixing part and the secondary fixing part are integrally formed by means of any one of stitching, hinging, crimping, ultrasonic welding, and high-frequency welding; and/or
at least two of the plurality of fixing straps are integrally formed by means of any one of stitching, hinging, crimping, ultrasonic welding, and high-frequency welding.

A second aspect of the present invention further provides a mask system configured with a headgear assembly provided in the first aspect of the present invention.

Compared with the prior art, the present application has the following advantages:
an embodiment of the present invention proposes a headgear assembly, including a primary fixing part and a secondary fixing part, the secondary fixing part being connected above the primary fixing part, the primary fixing part being configured to wrap around the back of the head of a human body, and the secondary fixing part being configured to wrap around the top of the head of the human body; where the primary fixing part includes: a plurality of fixing straps, at least two of the plurality of fixing straps being connected to each other, a free end of each of the fixing straps being provided with a connection point, and the connection point being configured for assembly to a mask system; the secondary fixing part includes: two top headstraps spaced side by side, with free ends of the two top headstraps being connected by a fixing structure, where the plurality of fixing straps include: an upper headstrap, an intermediate connecting strap, and a lower headstrap, two end portions of the intermediate connecting strap being respectively connected to a middle region of each of the upper headstrap and the lower headstrap.

By means of the technical solution of the present application, the primary fixing part can form a semi-enclosed cavity structure that wraps around and fits snugly against the back of the head of the human body when the primary fixing part is assembled with a mask of a mask system, so as to achieve initial positioning and fixation of the mask; and the two top headstraps in the secondary fixing part can be connected to form a fully enclosing structure that wraps around and fits snugly against the top of the head of the human body, such that after the mask is initially fixed, the wrapping between the top and the head is increased by means of the secondary fixing part to achieve enhanced fixation of the mask. In this way, the combination of the primary fixing part and the secondary fixing part can form a 3D wrapping effect without requiring 3D technology support, and is adaptively applicable to mask systems with or without a forehead support, so as to achieve better head wrapping, simplicity of manufacturing, and a wide scope of application at a low cost. Moreover, the feature of being adjustable at a plurality of points better achieves wearing comfort.

Since the above-described headgear assembly has the above technical effects, the mask system with the headgear assembly provided in the embodiments of the present invention should have similar technical effects. Details are not described herein.

The above description is merely a summary of the technical solutions of the present application. To make the technical means of the present application more clearly understood and implemented according to the contents of the specification, and to make the above and other objectives, features, and advantages of the present application more obvious and comprehensible, the specific embodiments of the present application are described in detail below.

### Brief Description of the Drawings

In order to more clearly illustrate the technical solutions in the present application, the drawings used in the description of the present application will be briefly described below. Apparently, the drawings in the following description are only some embodiments of the present application, and a person of ordinary skill in the art can obtain other drawings according to the drawings without any creative effort.
Fig. 1 is a schematic structural diagram of an "I-shaped" headgear assembly in the prior art;
Fig. 2 is a schematic structural diagram of a "crown-shaped" headgear assembly in the prior art;
Fig. 3 is a schematic diagram showing an unfolded structure of a headgear assembly according to an embodiment of the present application; and
Fig. 4 is a schematic diagram showing an unfolded structure of a headgear assembly according to another embodiment of the present application.

List of reference signs:
1. First top headstrap; 2. Second top headstrap; 3. Upper headstrap; 4. Lower headstrap; 5. Left headstrap; 6. Right headstrap; 7. Fixed connector; 8. Adjustable connector; 9. Connection point.

### Detailed Description of Embodiments

The technical solutions in the embodiments of the present application will be clearly and completely described below in conjunction with the accompanying drawings in the embodiments of the present application. Apparently, the embodiments described are some rather than all of the embodiments of the present application. All other embodiments obtained by those of ordinary skill in the art based on the embodiments of the present application without creative efforts shall fall within the scope of protection of the present application.

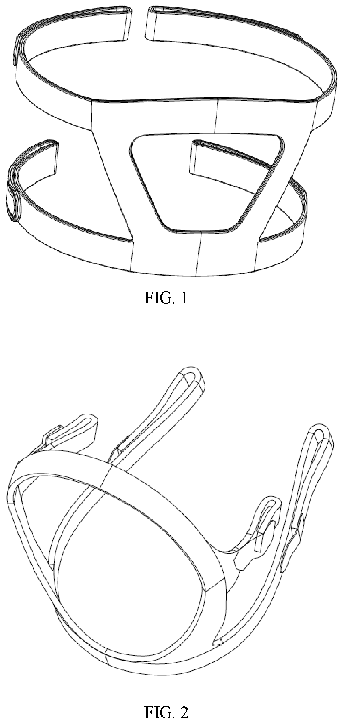

Figs. 1 and 2 respectively show schematic structural diagrams of an "I-shaped" headgear assembly and a "crown-shaped" headgear assembly in the prior art. The "I-shaped" headgear assembly is connected to a mask of a mask system via four connection points, and includes an upper headstrap and a lower headstrap that are arranged nearly parallel, where two ends of each of the upper headstrap and the lower headstrap serve as two connection points for connection to the mask, and a middle part of each of the upper headstrap and the lower headstrap is integrally connected by a longitudinal connecting strap, forming a configuration similar to the word "I". As can be seen, the upper headstrap may be connected to a forehead support of the mask during use, and the lower headstrap is connected to a mouth and nose support of the mask, but it cannot fix the top of the user's head, which does not form a 3D enveloping effect and results in low wearing firmness, and there are few suitable types of masks, which has great limitations.

The "crown-shaped" headgear assembly is connected to a mask of a mask system via four connection points, and includes an upper headstrap and a lower headstrap arranged nearly parallel, the upper headstrap and the lower headstrap being respectively located on an elliptical connecting strap fitting the back of the user's head. Because the elliptical connecting strap is shaped to fit on the top of the user's head, it improves wearing firmness compared with the "I-shaped" headgear assembly, providing positioning over the head of the wearer, which can generally be used for a mask system with a forehead support or for a mask system without a forehead support. However, in the process of building a fit to the top of the user's head, due to its special curvature shape design, it requires a large amount of clinical data on the top of the head to carry out 3D modeling, making the manufacturing process complex. The process is time-consuming and costly, and for some users with special head shapes or sizes, a reasonable 3D shape is extremely difficult to build.

The structure of the forehead support and the mouth and nose support varies depending on the structure of the mask. Thus, the forehead support referred to herein may be understood as a part of the mask that faces the forehead position of the user, and the mouth and nose support may be understood as another part of the mask that faces the mouth and nose position of the user.

It can be seen that the existing headgear assembly cannot accommodate many aspects such as the wide range of applicable mask types, simple and easy production process, high wearing firmness, and high applicability to individual differences. There is still much room for improvement in the user experience when it comes to a client side.

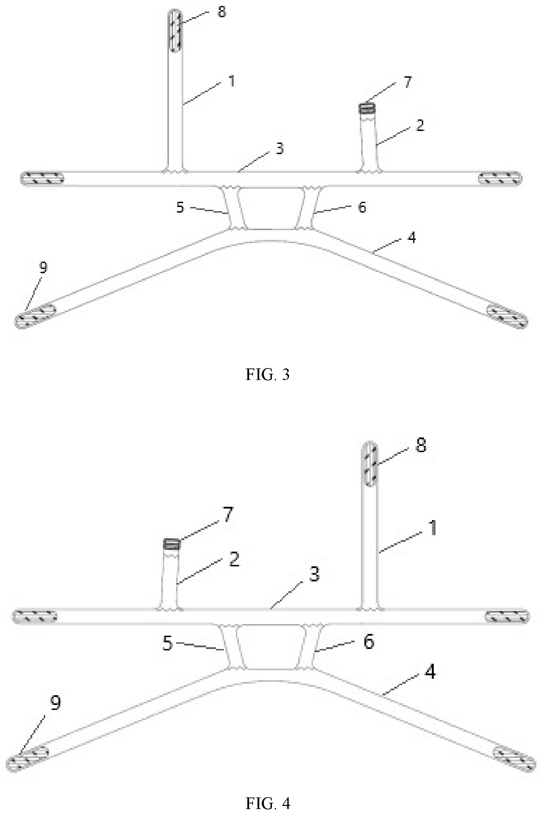

In view of this, the present invention aims to provide a headgear assembly, and as shown in Figs. 3 and 4, exemplary headgear assemblies according to some embodiments of the present application are shown in Figs. 3 and 4, respectively. The headgear assembly includes a primary fixing part and a secondary fixing part, the secondary fixing part being connected above the primary fixing part, the primary fixing part being configured to wrap around the back of the head of a human body, and the secondary fixing part being configured to wrap around the top of the head of the human body; where the primary fixing part includes: a plurality of fixing straps, at least two of the plurality of fixing straps being connected to each other, a free end of each of the fixing straps being provided with a connection point 9, and the connection point 9 being configured for assembly to a mask system; the secondary fixing part includes: two top headstraps spaced side by side, with free ends of the two top headstraps being connected by a fixing structure, and the plurality of fixing straps including: an upper headstrap, an intermediate connecting strap, and a lower headstrap, two end portions of the intermediate connecting strap being respectively connected to a middle region of each of the upper headstrap and the lower headstrap.

Specifically, both the primary fixing part and the secondary fixing part are configured to position and secure the mask of the mask system in a relatively comfortable position on the face, ensuring that the mask does not loosen or fall off during use, thereby providing a good seal and achieving the conditioning, improvement and therapeutic functions of the mask system. The primary fixing part and the secondary fixing part are arranged from the bottom to the top of the head of the human body. The primary fixing part is designed to surround the back of the head of the human body, and the secondary fixing part is designed to surround the top of the head of the human body to achieve a 3D wrapping effect of the head.

In particular, the plurality of fixing straps in the primary fixing part refer to straps whose positions, structures, shapes, and sizes are partially or completely different, but which function to connect the mask to the head of the human body. At least two fixing straps are connected to each other, which can refer to: all ends of one or more fixing straps are connected to the ends or bodies of other fixing straps, and all or some of the other fixing straps are not connected; and can also refer to: some ends of one or more fixing straps are connected to the ends or bodies of other fixing straps, and the remaining ends are not connected to the other fixing straps. Thus, the plurality of fixing straps may form a continuous semi-enclosed structure that wraps around the back of the head of the human body, which is a support point for securing the mask to the human face.

In this embodiment, each fixing strap has a free end and a connection point 9, where the free end may refer to an end of the plurality of connected fixing straps that is not fixed or connected, and corresponds to a fixed end, the fixed end being a position at which the plurality of connected fixing straps are connected. As a specific explanation, two ends of one fixing strap are respectively connected to one end of each of the other two fixing straps, and all ends of this fixing strap are fixed ends, and the ends of the other two fixing straps that are connected are fixed ends and the other ends are free ends.

The free end typically has a specific structure or feature, and in this embodiment of the present invention, a connection point 9 is provided on the free end, which can be adjusted, connected or fixed with other components (such as a mask) to fit the shape and size of the patient's head, thereby initially securing the mask on the back of the head of the human body. It should be understood that in this implementation, there are fixed points on the mask system that match the connection points 9, and the two have a one-to-one correspondence. In some embodiments, the connection point 9 on the primary fixing part may be fitted in a snap-in, snap-on, hook-on, or other type of assembly to the fixed point on the mask system. This may depend on the design of the mask system and is not specifically limited in this document.

Accordingly, it will also be appreciated that the primary fixing part of the headgear assembly of the present invention may be used independently of the secondary fixing part and may be applicable to a mask system with a forehead support.

More specifically, the two top headstraps are connected to one of the plurality of fixing straps in the primary fixing part that is closest to the top headstrap such that the secondary fixing part is located above the primary fixing part. The side-by-side arrangement of the two top headstraps may be understood as being connected to the same side of the corresponding fixing strap and having a distance between the two. In some embodiments, the lengths of the two top headstraps may be the same or different. In some embodiments, the widths of the two top headstraps may be the same or different. In some embodiments, the width of at least one top headstrap is a uniform width or gradient width, and the top headstrap having a gradient width may gradually increase or decrease in width from the direction approaching the corresponding fixing strap to the outside.

Two end portions of the intermediate connecting strap are respectively connected to the middle region of each of the upper headstrap 3 and the lower headstrap 4, and it can be understood that the upper headstrap 3 and the lower headstrap 4 are of an upper and lower split structure, with the middle portion being integrally formed by means of the intermediate connecting strap. The distance between the upper headstrap 3 and the lower headstrap 4 is equal to a vertical strap length of the intermediate connecting strap. In some embodiments, the intermediate connecting strap may be upright, that is, the distance between the upper headstrap 3 and the upper headstrap 4 is a strap length of the intermediate connecting strap. In some embodiments, the intermediate connecting strap may be inclined, that is, the distance between the upper headstrap 3 and the lower headstrap 4 is a vertical length of the strap length of the intermediate connecting strap.

In this implementation, the upper headstrap 3 and the lower headstrap 4 are respectively located at different heights in the back of the head of the human body, and the weight of the head is evenly distributed, so as to reduce the stress of the headstrap on the head and improve wearing comfort. The upper headstrap 3 and the lower headstrap 4 can be adjusted separately when the headstrap is worn or removed to adjust the length and tightness of the headstrap for different users and different head structures. It also makes it easier to wear and remove the headstrap without interference from the user's hair or ears.

Specifically, in the upper and lower split structure, the shapes and structures of the upper headstrap 3 and the lower headstrap 4 can be arranged respectively under the condition of maintaining head balance. For example, the upper headstrap 3 may have a horizontal strap structure, which allows it to extend smoothly along an outer edge of an upper part of the back of the head of the human body, and the lower headstrap 4 may have an arc-shaped strap structure, which allows it to extend along a lower part of the back of the head of the human body toward the neck region of the human body, thereby increasing the diversity and selectivity of the headstrap.

In some embodiments, the two top headstraps are connected by means of a fixing structure, so that two ends of each of the two top headstraps are connected to each other, thereby forming a fully enclosing structure that wraps around and fits snugly against the top of the head of the human body. For a mask system without a forehead support, when the primary fixing part is initially fixed, the secondary fixing part increases the wrapping between the top and the head to enhance the fixation of the mask. The two parts thus wrap the specific parts of the head separately to create a 3D wrapping effect that fits both the mask system with a forehead support and the mask system without a forehead support and is secure and comfortable to wear.

Thus, when facing the client side, there is no need to collect a large amount of user head data, allowing for consideration of head features for different users, and it is comfortable and secure to wear, preventing the mask from moving or loosening, improving protection or therapeutic effectiveness, and meeting different medical scenarios or user needs, thereby enhancing the wearing experience. Compared with a 3D crown headgear, it is simpler to produce and has lower costs, providing more options for users with sleep and/or respiratory conditions as well as a platform side.

In some embodiments, the plurality of fixing straps in the primary fixing part and the two top headstraps in the secondary fixing part each have a planar feature. The planar feature can be understood as the fixing strap and the top headstrap being generally planar, made from banded fabric, rubber, polymer, plastic or leather, etc., and being relatively flat horizontally, without complex curves or a three-dimensional effect. Also, the headgear assembly is relatively even in thickness and is evenly distributed throughout the height direction with no prominent protrusion, depression or variation. Flat fixing straps and flat top headstraps allow the headgear assembly to provide a uniform contact surface when in contact with the user's head, reducing the concentration of local pressure and making the headgear more stable and comfortable to wear.

The integral flat primary fixing part and secondary fixing part work together to create a 3D wrapping effect and fit the head well. It can be understood that flat modeling makes it easier to design, make molds, and implement production processes than the 3D crown headgear. In some embodiments, a planar headgear assembly may be manufactured by conventional simple splicing, molding, and connecting processes, thereby reducing raw material costs. It also eliminates the need for complex molds and production equipment, and the production process is simple and easy, the material utilization is high, and the total cost of expenditure is significantly reduced.

In some embodiments, the fixing structure includes a fixed connector 7 and an adjustable connector 8 that cooperate with each other. The fixed connector 7 is disposed on one of the two top headstraps, and the adjustable connector 8 is disposed on the other top headstrap. In this embodiment, the fixed connector 7 can be understood as providing a fixed support point for size-adjustable connection of the adjustable connector 8, and the relative position between the fixed connector 7 and the corresponding headgear assembly remains unchanged; and the adjustable connector 8 is mounted on the other top headstrap, and the relative position between the adjustable connector and the corresponding headgear assembly is changeable. In this way, the size of the fully enclosing structure formed by the two top headstraps connected together can be changed to adjust the tightness of the secondary fixing part to fit different head sizes. It is worth mentioning that the connection point 9 of the fixing strap may be of the same structure as the adjustable connector 8 of the top headstrap and mate with a fixing point on the mask, and the fixing point on the mask may adopt the structure of the fixed connector 7 of the present application, thereby adjusting the tightness of the primary fixing part to fit different head sizes. In this way, the feature of being adjustable at a plurality of points allows for better wearing comfort.

In some embodiments, the fixed connector 7 may be a hook fastener, a buckle, a button, a snap ring or the like, which is fixed on the corresponding top headstrap. In some embodiments, the adjustable connector 8 may be a retractable strap, a stretchable elastic strap, or a strap having adjustment holes, that cooperates with the fixed connector 7, such that a user can fix the adjustable connector 8 at different positions or holes in the fixed connector 7, so as to achieve customized adjustment of the headstrap. In some examples derived from this embodiment, one of the top headstraps can serve as the adjustable connector 8, that is, the top headstrap has an adjustable length or stretchability, and the top headstrap is fixed to another top headstrap having the fixed connector 7. In some embodiments, the adjustable connector 8 may be a hook-and-loop fastener that engages with the fixed connector 7 and is bonded to the corresponding top headstrap. It is worth mentioning that the material of each fixing strap and the material of the top headstrap may be the same and both are stretchable and elastic.

More precisely, the fixed connector 7 includes a buckle, and the adjustable connector 8 includes a hook-and-loop fastener. In this embodiment, the buckle may be a tri-glide adjustable buckle formed by a strap webbing having two apertures and a buckle ring having a fixed stud. One of the apertures is configured to allow for one of the top headstraps to pass therethrough and be secured to this top headstrap, and the other aperture is configured to allow for the other top headstrap to pass therethrough. The other top headstrap is provided with a hook-and-loop fastener that may be formed by a soft loop layer and a rough hook layer horizontally splicing together, such that when the loop layer comes into contact with the hook layer, the hooks can engage with the loops. In some embodiments, the loop layer and the hook layer may be disposed on the same side of the corresponding top headstrap, and are located in different regions in a direction of strap length of the top headstrap. When an end portion of the top headstrap having the loop layer or the hook layer passes through the other aperture of the buckle, the end portion of the top headstrap folds back and then comes into contact with its own hook layer or loop layer, allowing the two top headstraps to connect to enclose a fully enclosing structure.

In some embodiments, the hook layer is closer to the end portion of the top headstrap than the loop layer, and the area of the hook layer is less than the area of the loop layer, such that the size of the fully enclosing structure is adjusted by pulling the end portion of the top headstrap to select the folding length. In some embodiments, the hook-and-loop fastener formed by the hook layer and the loop layer is entirely bonded to a surface of the top headstrap facing away from the head of a human body. In some embodiments, a plurality of loop layers arranged at intervals may be provided along a strap length of the top headstrap, the area of each loop layer may be less than, slightly greater than, or equal to an area of the hook layer, and the size of the fully enclosing structure is adjusted by selecting the hook layer to come into contact with the loop layers at different positions. In some embodiments, the hook layer may have a gap with the end portion of the top headstrap.

In this way, through the cooperation of the buckle and the hook-and-loop fastener, the two top headstraps can be securely wrapped around the head region, and the folding length of the top headstrap provided with the hook-and-loop fastener can be adjusted as needed, so as to achieve a customized wearing experience to provide better comfort, fittability and adjustability and ensure that the headstraps play a role of enhanced fixation during use.

As a specific explanation of this embodiment, the two top headstraps have different strap lengths, and the fixed connector 7 is disposed on the top headstrap having a shorter strap length. Specifically, the two top headstraps having different strap lengths can be understood as that the distances the top headstraps extend outward from their connection with the fixing strap are different. In a case where the strap length of the first top headstrap 1 is greater than the strap length of the second top headstrap 2, the first top headstrap 1 and the second top headstrap 2 are both disposed on the corresponding fixing strap. In some embodiments, the distance between the first top headstrap 1 and the adjacent free end of the fixing strap may be less than or equal to the distance between the second top headstrap 2 and the adjacent free end of the fixing strap. In some embodiments, the distance between the first top headstrap 1 and the adjacent free end of the fixing strap may be greater than the distance between the second top headstrap 2 and the adjacent free end of the fixing strap. In some embodiments, a free end of the first top headstrap 1 is provided with a buckle, and a free end of the second top headstrap 2 is provided with a hook-and-loop fastener. In some embodiments, the free end of the first top headstrap 1 is provided with a hook-and-loop fastener, and the free end of the second top headstrap 2 is provided with a buckle.

It should be understood that the distance between the first top headstrap 1 and the second top headstrap 2 is such that when the fixing strap is assembled onto the mask, the first top headstrap 1 and the second top headstrap 2 can be respectively located on the left and right sides of the head of the human body, rather than on the same side.

In a preferred embodiment, the first top headstrap 1 and the second top headstrap 2 are symmetrically disposed with respect to a centerline of the corresponding fixing strap, that is, respectively located on the left and right sides of the corresponding fixing strap. The hook-and-loop fastener is located on the first top headstrap 1, and the buckle is located on the second top headstrap 2. This allows for the first top headstrap 1, which has a longer strap length, to have a longer folding range after passing through the aperture of the buckle, so as to meet the requirements to achieve more precise fixation and fit different head sizes by selecting the contact position between the hook layer and the loop layer. The second top headstrap 2, which has a shorter strap length, is less prone to shaking or swinging during a positioning process, facilitating a user to quickly and smoothly pass the first headgear assembly through the aperture of the buckle.

In an optional embodiment, referring to Fig. 3 again, the first top headstrap 1 is located on the left portion of the corresponding fixing strap, and the free end of the first top headstrap 1 is provided with a hook-and-loop fastener; and the second top headstrap 2 is located on the right portion of the same fixing strap, and the free end of the second top headstrap 2 is provided with a buckle. The head assembly facilitates use by left-handed users, who can grasp the first top headstrap 1 having the hook-and-loop fastener with their left hand and stabilize the second top headstrap 2 having the buckle with their right hand, and insert the first top headstrap 1 into the aperture of the buckle and fold it back until the hook layer comes into contact with its own loop layer.

In another optional embodiment, referring to Fig. 4 again, the first top headstrap 1 is located on the right portion of the corresponding fixing strap, and the free end of the first top headstrap 1 is provided with a hook-and-loop fastener; and the second top headstrap 2 is located on the left portion of the same fixing strap, and the free end of the second top headstrap 2 is provided with a buckle. Such a headgear assembly facilitates use by right-handed users, who can grasp the first top headstrap 1 having the hook-and-loop fastener with their right hand and stabilize the second top headstrap 2 having the buckle with their left hand, and insert the first top headstrap 1 into the aperture of the buckle and fold it back until the hook layer comes into contact with its own loop layer.

In this way, the headgear assembly can meet the usage habits of different users, making a connection process more convenient and in line with individual preferences.

Consequently, in a case where the strap length of the first top headstrap 1 is less than the strap length of the second top headstrap 2, various types of configuration mentioned above are also applicable. This method has a wide variety of combinations and variations, which can meet various styling and shaping requirements of users for the headstrap.

In another embodiment, an angle formed between the first top headstrap 1 and/or the second top headstrap 2 of the two top headstraps and the fixing strap correspondingly connected thereto may be an acute angle, an obtuse angle, or a right angle. Preferably, an angle between each of the two top headstraps and the fixing strap correspondingly connected thereto ranges from 80° to 100°. In some embodiments, the angles between the two top headstraps and the fixing straps correspondingly connected thereto are equal.

In the connection between the top headstrap and the corresponding fixing strap, the corresponding fixing strap is an upper headstrap 3 in this embodiment. That is, an angle between each of the two top headstraps and the upper headstrap 3 ranges from 80° to 100°. In a preferred embodiment, the first top headstrap 1 and the second top headstrap 2 are symmetrically disposed with respect to the centerline of the upper headstrap 3, and are equidistant from an intermediate connecting strap. That is, the distance between the first top headstrap 1 and the left headstrap 5 may be equal to the distance between the second top headstrap 2 and the right headstrap 6.

In this embodiment, the angle refers to an angle on an inner side between the top headstrap and the corresponding fixing strap, and the inner side can be understood as a side proximate to a central region of a posterior side of the head of a human body. The top headstrap being at an acute angle means that the first top headstrap 1 and/or the second top headstrap 2 are arranged obliquely outward and extend inward from the connection with the fixing strap, which can form a relatively compact structure. The top headstrap being at an obtuse angle means that the first top headstrap 1 and/or the second top headstrap 2 are arranged obliquely outward and extend outward from the connection with the fixing strap, which can form a relatively loose structure. The top headstrap being at a right angle means that the first top headstrap 1 and/or the second top headstrap 2 extend vertically outward from the connection with the fixing strap and are perpendicular to the corresponding fixing strap.

In some embodiments, the angle between the first top headstrap 1 and the corresponding fixing strap and the angle between the second top headstrap 2 and the corresponding fixing strap may be different. In some embodiments, the angle between the first top headstrap 1 and the corresponding fixing strap and the angle between the second top headstrap 2 and the corresponding fixing strap may be equal.

In a preferred embodiment, the angle between the first top headstrap 1 and the corresponding fixing strap and the angle between the second top headstrap 2 and the corresponding fixing strap are equal and both are acute angles. In this implementation, the first top headstrap 1 and the second top headstrap 2 may be symmetrically distributed after being connected, such that the pressure around the head is balanced, so as to avoid applying excessive pressure or causing discomfort on specific parts, which is conducive to providing a more comfortable wearing experience. An acute-angled design reduces the gap between the headgear assembly and the head, which ensures that the headgear assembly fits snugly against the head and prevents displacement or loosening of the headgear assembly during use, thereby enhancing the overall performance and wearing experience of the headgear assembly.

This embodiment is used to illustrate the specific structure of a plurality of fixing straps in the primary fixing part.

The plurality of fixing straps include
an upper headstrap 3, an intermediate connecting strap, and a lower headstrap 4. Two end portions of the intermediate connecting strap are respectively connected to middle regions of the upper headstrap 3 and the lower headstrap 4. The intermediate connecting strap includes a left headstrap 5 and a right headstrap 6 arranged obliquely relative to each other, so as to jointly enclose a variable-diameter wrapping cavity together with the upper headstrap 3 and the lower headstrap 4.

Specifically, the upper headstrap 3, the intermediate connecting strap and the lower headstrap 4 are sequentially arranged from top to bottom. The upper headstrap 3 is configured to interconnect the intermediate connecting strap and the top headstraps. The intermediate connecting strap is configured to connect the upper headstrap 3 and the lower headstrap 4. The upper headstrap 3, the intermediate connecting strap and the lower headstrap 4 may enclose a semi-enclosed cavity structure configured to wrap around the back of the head of the human body, and the upper headstrap 3 and the two connected top headstraps may enclose a fully enclosing structure configured to wrap around the top of the head of the human body. In some embodiments, the upper headstrap 3 is a straight strip extending horizontally and transversely that can fit along the same height direction of the head of the human body until it is connected to the mask, exhibiting a feature of stable positioning, and facilitating the connection of the two top headstraps onto the strap of the upper headstrap 3. In some embodiments, the lower headstrap 4 may be arranged parallel to or at an angle to the upper headstrap 3.

Two ends of each of the left headstrap 5 and the right headstrap 6 are respectively connected to the straps of the upper headstrap 3 and the lower headstrap 4, so as to form a variable-diameter wrapping cavity. The left headstrap 5 and the right headstrap 6 are arranged obliquely relative to each other and are spaced apart by a distance, that is, they form an included angle or an inclination in a horizontal direction, and their directions of inclination are close to each other. The variable-diameter wrapping cavity can be understood as having different inner diameters or diameters in the height direction, with the area of the wrapping cavity varying from top to bottom. In some embodiments, the variable-diameter wrapping cavity is of an inverted trapezoid, that is, the left headstrap 5 and the right headstrap 6 extend obliquely outward from the lower headstrap 4 toward the upper headstrap 3, with the angles of inclination thereof being equal, such that the area of the wrapping cavity gradually increases from bottom to top to fit the curvature and shape variations of the back of the head of the human body, which can achieve a better fitting and wrapping effect, reduce the overall weight, and provide the users with a more comfortable wearing experience.

It should be understood that the variable-diameter wrapping cavity is typically located in the middle region of the entire headgear assembly, and the distance between one of the free ends of the upper headstrap 3 and/or the lower headstrap 4 and the central axis of the variable-diameter wrapping cavity and the distance between the other free end and the central axis may be selected within a wide range. Preferably, the two free ends of the upper headstrap 3 and/or the lower headstrap 4 are symmetrically arranged with respect to the central axis of the variable-diameter wrapping cavity, which is aesthetically pleasing and facilitates adjustment.

In some embodiments, the lengths of the upper headstrap 3 and the lower headstrap 4 may be the same or different, and the length of the intermediate connecting strap may be less than either of them. In some embodiments, the widths of the upper headstrap 3, the intermediate connecting strap, and the lower headstrap 4 may be the same or different. In some embodiments, the width of at least one of the upper headstrap 3, the intermediate connecting strap and the lower headstrap 4 is a uniform width or a gradient width. The gradient width of at least one of them may gradually increase or decrease from bottom to top.

In a preferred embodiment, the lower headstrap 4 and the upper headstrap 3 are arranged in a non-parallel manner. Specifically, the upper headstrap 3 may be horizontally arranged, and the lower headstrap 4 curves toward the neck region of the human body to form an arc-shaped opening, and an angle of the arc-shaped opening is an obtuse angle. In this embodiment, the lower headstrap 4 is bent at a central region thereof to form an inverted V-shaped structure with an opening facing the neck of the human body. An angle of the opening is an obtuse angle, facilitating two mutually symmetrical sides of the V-shaped lower headstrap 4 to extend obliquely downward toward the neck of the human body, and can thus go around behind the ears when assembled with the mask, thereby reaching the neck region of the human body to achieve a 3D wrapping effect.

In another embodiment, the primary fixing part and the secondary fixing part are integrally connected by any one of stitching, hinging, crimping, ultrasonic welding, and high-frequency welding, and/or at least two of the plurality of fixing straps are integrally connected by any one of stitching, hinging, crimping, ultrasonic welding, and high-frequency welding.

Specifically, as mentioned above, each of the fixing straps and the top headstrap has a free end and a fixed end. The fixed ends are locations where a plurality of fixing straps are connected to each other and locations where the corresponding fixing straps are connected to the top headstrap. The first top headstrap 1 and the second top headstrap 2 may be connected together with the upper headstrap 3 in various ways to form a fixed end; the first top headstrap 1 or the second top headstrap 2 may be connected together with the fixed connector 7 in various ways to form a fixed end; the right headstrap 6, the left headstrap 5 and the upper headstrap 3 may be connected together in various ways to form a fixed end; and the right headstrap 6, the left headstrap 5 and the lower headstrap 4 may be connected together in various ways to form a fixed end. The connection methods mentioned above have different characteristics and scopes of application, and the most suitable connection method can be selected according to specific materials, design requirements and manufacturing processes. In this embodiment, the connection methods for the fixed ends may be partially or completely the same, or completely different.

It should be noted that the stitching may refer to stitching edges or contact portions of two connecting components (hereinafter exemplified by the upper headstrap 3 and the first top headstrap 1) together. For example, an end portion of the first top headstrap 1 is stitched to a right portion of the upper headstrap 3, so as to form a continuous headstrap structure by stitching their edges together;
the hinging may be achieved by mounting a hinge or the like onto the upper headstrap 3 and the first top headstrap 1, enabling them to rotate or swing relative to each other;
the crimping may refer to tightly pressing contact surfaces of the upper headstrap 3 and the first top headstrap 1 together, so as to create a connection under the action of pressure and friction to form a firm fixed end;
the ultrasonic welding may refer to using thermal energy generated by ultrasonic vibration to heat and fuse the contact surfaces of the upper headstrap 3 and the first top headstrap 1 together to form a strong fixed end; and
the high-frequency welding may refer to using thermal energy generated by a high-frequency electromagnetic field to heat and fuse the contact surfaces of the upper headstrap 3 and the first top headstrap 1 together to form a sturdy fixed end.

Consequently, the upper headstrap 3 and the first top headstrap 1 may also be connected and formed by other connection processes, and the specific connection processes and methods may vary depending on the materials or shapes of the upper headstrap 3 and the first top headstrap 1. The above examples are only to illustrate the functions and principles of different connection methods. This can be flexibly selected in practical applications.

In summary, the primary fixing part can form a semi-enclosed structure that wraps around and fits snugly against the back of the head of the human body when the primary fixing part is assembled with a mask of a mask system, so as to achieve initial positioning and fixation of the mask; and the two top headstraps in the secondary fixing part can be connected to form a fully enclosing structure that wraps around and fits snugly against the top of the head of the human body, such that after the mask is initially fixed, the wrapping between the top and the head is increased by means of the secondary fixing part to achieve enhanced fixation of the mask. In this way, the combination of the primary fixing part and the secondary fixing part can form a 3D wrapping effect without requiring 3D technology support, and is adaptively applicable to mask systems with or without a forehead support, so as to achieve better head wrapping, simplicity of manufacturing, and a wide scope of application at a low cost. Moreover, the feature of being adjustable at a plurality of points better achieves wearing comfort. This method has a wide variety of combinations and variations, which can meet various styling and shaping requirements of users for headstraps, so as to provide a better head fixation solution in the field of ventilation therapy.

Another aspect of the present invention is to provide a mask system configured with a headgear assembly described above.

The aforementioned mask system embodiments are basically similar to the headgear assembly embodiments, so the description is relatively simple. For relevant details, reference can be made to the description of the headgear assembly embodiments.

It should be noted that the embodiments in this specification are described in a progressive manner, each embodiment focuses on the difference from other embodiments, and for the same or similar parts of the embodiments, reference can be made to each other.

It should further be noted that the orientation or positional relationships indicated by the terms "upper", "lower", "left", "right", "inner", "outer", etc., herein are based on the orientation or positional relationship shown in the accompanying drawings and are only for facilitating the description of the present invention and simplifying the description, rather than indicating or implying that an apparatus or an element referred to must have a particular orientation or be constructed and operated in a particular orientation, and therefore will not be interpreted as limiting the present invention. In addition, relationship terms such as "first" and "second" are merely used to distinguish one entity or operation from another, without necessarily requiring or implying any such actual relationship or order between these entities or operations, nor should they be construed as indicating or implying relative importance. Moreover, the terms "include" or any other variations thereof are intended to cover non-exclusive inclusion, so that a process, method, article, or terminal device that includes a series of elements not only includes those elements but also includes other elements not specifically listed, or elements inherent to such a process, method, article, or terminal device.

The headgear assembly and the mask system provided in the present application are described in detail above. Specific examples are applied herein to elaborate on the principles and implementations of the present application. The descriptions of the above embodiments are merely used to help understanding the present application, and the content of this specification should not be construed as a limitation on the present application. In addition, for those ordinary skilled in the art, various forms of modifications may be made to the specific implementations and scope of application based on the present application, and it is neither necessary nor possible to exhaustively list all implementations herein, and any obvious changes or variations derived therefrom shall still fall within the protection scope of the present application.

## Claims

1. A headgear assembly, **characterized by** comprising a primary fixing part and a secondary fixing part, the secondary fixing part being connected above the primary fixing part, the primary fixing part being configured to wrap around the back of the head of a human body, and the secondary fixing part being configured to wrap around the top of the head of the human body; wherein
the primary fixing part comprises:
a plurality of fixing straps, at least two of the plurality of fixing straps being connected to each other, and a free end of each of the fixing straps being provided with a connection point for assembly to a mask system; and
the secondary fixing part comprises:
two top headstraps spaced side by side, with free ends of the two top headstraps being connected by a fixing structure;
wherein the plurality of fixing straps comprise:
an upper headstrap, an intermediate connecting strap, and a lower headstrap, two end portions of the intermediate connecting strap being respectively connected to a middle region of each of the upper headstrap and the lower headstrap.

2. The headgear assembly according to claim 1, **characterized in that** the fixing structure comprises a fixed connector and an adjustable connector cooperating with each other, the fixed connector being disposed on one of the two top headstraps, and the adjustable connector being disposed on the other of the two top headstraps.

3. The headgear assembly according to claim 2, **characterized in that** the two top headstraps have different strap lengths, and the fixed connector is disposed on the top headstrap with a shorter strap length.

4. The headgear assembly according to claim 2 or 3, **characterized in that** the fixed connector comprises a buckle, and the adjustable connector comprises a hook-and-loop fastener.

5. The headgear assembly according to any one of claims 1-4, **characterized in that** an angle between each of the two top headstraps and the fixing strap correspondingly connected thereto ranges from 80° to 100°.

6. The headgear assembly according to any one of claims 1-5, **characterized in that** angles between the two top headstraps and the fixing straps correspondingly connected thereto are equal.

7. The headgear assembly according to any one of claims 1-6, **characterized in that** the intermediate connecting strap comprises a left headstrap and a right headstrap arranged obliquely relative to each other, and the left headstrap and the right headstrap together with the upper headstrap and the lower headstrap enclose a variable-diameter wrapping cavity.

8. The headgear assembly according to any one of claims 1-7, **characterized in that** the lower headstrap curves toward a neck region of the human body to form an arc-shaped opening with an obtuse angle.

9. The headgear assembly according to any one of claims 1-8, **characterized in that** the plurality of fixing straps and the two top headstraps have planar features.

10. The headgear assembly according to any one of claims 1-9, **characterized in that** the two top headstraps are connected to the upper headstrap, and are equidistant from the intermediate connecting strap.

11. The headgear assembly according to any one of claims 1-10, **characterized in that** the primary fixing part and the secondary fixing part are integrally formed by means of any one of stitching, hinging, crimping, ultrasonic welding, and high-frequency welding; and/or
at least two of the plurality of fixing straps are integrally formed by means of any one of stitching, hinging, crimping, ultrasonic welding, and high-frequency welding.

12. A mask system configured with a headgear assembly according to any one of claims 1-11.
